# EUROPEAN PATENT APPLICATION

(11) **EP 4 799 633 A1**
(43) Date of publication of application: **02.09.2026**
(21) Application number: 25382185.4
(22) Date of filing: 28.02.2025
(51) Int. Cl.: A61K 35/747, A61K 9/00, A61P 15/02, A61P 15/06, A61P 15/08, A61P 31/00, A61Q 90/00

(54) **LACTOBACILLUS CRISPATUS STRAIN FOR IMPROVEMENT OF GENITAL MICROBIOTA**

(71) Applicant: Kaneka Corporation, Osaka-shi, Osaka 530-8288 (JP); AB-Biotics, S.A., 08174 Sant Cugat del Vallès Barcelona (ES)
(72) Inventor: YAURA, Hisako, Hyogo, 676-8688 (JP); HONDA, Shinichi, Hyogo, 676-8688 (JP); PÉREZ GARCÍA, Marta, 08174 Barcelona (ES); HUEDO MORENO, Pol, 08174 Barcelona (ES); ARMENGOL RIVERO, Eva, 08174 Barcelona (ES); ESPADALER MAZO, Jordi, 08174 Barcelona (ES)
(74) Representative: Hoffmann Eitle

(57) **Abstract**

A problem to be addressed by the present invention is to provide a new genus *Lactobacillus* bacterium that has a glycogen-assimilating ability and can inhibit the proliferation of various kinds of pathogens. The present invention provides a *Lactobacillus crispatus* strain deposited under accession No. CECT31133.

## Description

### BACKGROUND OF THE INVENTION

### Field of the Invention

The present invention relates to: a *Lactobacillus crispatus* strain suitable for improvement of a genital microbiota; and a composition comprising the *Lactobacillus crispatus* strain.

### Description of the Related Art

At present, a declining birth rate is a social problem in East Asian countries, such as Japan, and European Countries, such as Spain and Italy. One of the factors causing this problem is a tendency to marry later. It is known that a fertility rate is decreased with advancing age (Non-Patent Document 1). Thus, there is a demand for means for increasing a fertility rate and a live birth rate.

The fertility rate and the live birth rate are associated with a vaginal microbiota. It is known that females having a microbiota containing many genus *Lactobacillus* bacteria have not only a high fertility rate but also a low stillbirth rate or abortion rate and a high live birth rate (Non-Patent Document 1 and 2).

The genus *Lactobacillus* bacterium is one kind of lactic acid bacterium. Lactic acid produced by this bacterium keeps the intravaginal pH usually at 3.8 or more and less than 4.5, thus inhibiting the invasion and proliferation of miscellaneous bacteria. In a vagina containing less genus *Lactobacillus* bacteria, the intravaginal pH becomes 4.5 or more, and causes miscellaneous bacteria to proliferate, thus triggering the onset of vaginitis, such as bacterial vaginosis. In the cases where vaginitis is left untreated, a bacterial infection may spread to the surrounding tissues and organs, and induce endometritis, salpingitis, and the like, leading to infertility (Non-Patent Document 3).

Accordingly, for treatment or prevention of an infection of a genital organ, for enhancement of fertility, and for enhancement of a live birth rate, it is important to control the vaginal microbiota so that it has good bacteria of genus *Lactobacillus* dominantly, and to control the intravaginal pH so that it falls within a normal range.

Patent Document 1 describes use of a specific *Lactobacillus crispatus* (*L. crispatus*) strain for prevention or treatment of a vaginal infection, improvement of fertility, or to decrease the risk of premature delivery. However, there is still a demand for a search for a new genus *Lactobacillus* bacterium having characteristics advantageous for improvement of the vaginal microbiota.

### Related Art Documents

### Patent Document

Patent Document 1: JP2024-503366A

### Non-Patent Document

Non-Patent Document 1: Nayara S. et al., 2022, Frontiers in Reproductive Health, vol. 3, 780931
Non-Patent Document 2: Agnese Maria Chiara Rapisarda et al., 2023, Frontiers in Surgery, 9: 1075612
Non-Patent Document 3: Jacques Ravel et al., 2021, American Journal Obstetrics & Gynecology, 224(3): 251-257

### SUMMARY OF THE INVENTION

A vaginal epithelial cell has glycogen accumulated through the action of an estrogen hormone. The turnover of the epithelial cell causes the glycogen to be eluted. Because of this, a genus *Lactobacillus* bacterium, which proliferates using glycogen as a nutrient source, is advantageous to be applied intravaginally. In addition, a genus *Lactobacillus* bacterium which can inhibit the proliferation of various pathogens is advantageous in improvement of a vaginal microbiota. Accordingly, a problem to be addressed by the present invention is to provide a new genus *Lactobacillus* bacterium that has a glycogen-assimilating ability, and can inhibit the proliferation of various kinds of pathogens.

The present inventors have made studies vigorously to solve the above-described problems, and consequently have come to complete the present invention through the discovery of a *Lactobacillus crispatus* strain that proliferates using glycogen as a nutrient source, can decrease the surrounding pH, and can inhibit the proliferation of pathogens that induce an infection of a genital organ.

That is, the present invention encompasses the following.
[1] A *Lactobacillus crispatus* strain deposited under accession No. CECT31133.
[2] A composition comprising a living bacterium of the *Lactobacillus crispatus* strain according to [1].
[3] The composition according to [2], for use in administration to a genital organ.
[4] The composition according to [2] or [3], for use in improvement of a genital microbiota.
[5] The composition according to any one of [2] to [4], for use in vaginal cleaning.
[6] The composition according to any one of [2] to [5], for use in prevention or treatment of an infection.
[7] The composition according to any one of [2] to [6], for use in improvement of fertility.
[8] The composition according to any one of [2] to [7], for use in decreasing a risk of premature delivery.

The *Lactobacillus crispatus* strain according to the present invention proliferates using glycogen as a nutrient source, and can decrease the surrounding pH. The *Lactobacillus crispatus* strain according to the present invention can also inhibit the proliferation of pathogens that induce an infection of a genital organ such as *E. coli, S. aureus, P. mirabilis, S. agalactiae, C. albicans, G. vaginalis,* and *P. bivia.*

### BRIEF DESCRIPTION OF THE DRAWINGS

FIG. 1 is a graph showing the proliferative abilities of 14 *L. crispatus* strains (KABP-067, Comparative Strains 1, 2, 3, 5, 6, 7, 12, 17, 18, 20, 21, 23, and 24) in a glucose-containing MRS medium;
FIG. 2 is a graph showing the proliferative abilities of the 14 *L. crispatus* strains (KABP-067, Comparative Strains 1, 2, 3, 5, 6, 7, 12, 17, 18, 20, 21, 23, and 24) in a glycogen-containing MRS medium;
FIG. 3 is a graph showing the pH-decreasing abilities of the 14 *L. crispatus* strains (KABP-067, Comparative Strains 1, 2, 3, 5, 6, 7, 12, 17, 18, 20, 21, 23, and 24) in a glucose-containing MRS medium; and
FIG. 4 is a graph showing the pH-decreasing abilities of the 14 *L. crispatus* strains (KABP-067, Comparative Strains 1, 2, 3, 5, 6, 7, 12, 17, 18, 20, 21, 23, and 24) in a glycogen-containing MRS medium.

### DETAILED DESCRIPTION OF THE INVENTION

The present invention will be described in detail below.

### 1. Lactobacillus crispatus strain

### 1-1. Outline

The present invention relates to a *Lactobacillus crispatus* (*L. crispatus*) strain that has an excellent glycogen-assimilating ability and can inhibit proliferation of a pathogen.

### 1-2. Constitution

The *Lactobacillus crispatus* strain according to the present invention is, in particular, *Lactobacillus crispatus* KABP-067 strain. The *Lactobacillus crispatus* KABP-067 strain has been internationally deposited with the Spanish Type Culture Collection, "Colección Española de Cultivos Tipo" (CECT) (Edificio 3 CUE. Parc Cientific Universitat de Valencia, Catedratico Agustin Escardino, 9, 46980 Paterna, Valencia), under accession No. CECT31133, as of September 24, 2024, under the Budapest Treaty.

As demonstrated in the below-described Examples, the KABP-067 strain (KABP^{™}-067 strain) was isolated for the first time from the vagina of Italian females by the present inventors.

The KABP-067 strain proliferates under mild acidity (for example, a pH of 3.0 to 6.0, 4.0 to 5.0, or 4.0 to 4.5), and can decrease the surrounding pH.

In addition, the KABP-067 strain has an excellent glucose-assimilating ability. As used herein, the "glucose-assimilating ability" (glucose-utilizing ability) of a lactic acid bacterium means the ability of the lactic acid bacterium to proliferate using glucose as a nutrient source. When lactic acid bacteria have proliferated, lactic acid produced by the lactic acid bacteria decreases the surrounding pH. Accordingly, the glucose-assimilating ability of a lactic acid bacterium such as the KABP-067 strain can be evaluated on the basis of the number and pH of the lactic acid bacteria in a liquid culture obtained by culturing the lactic acid bacteria in a glucose-containing medium. For example, in a case where lactic acid bacteria are cultured in an MRS (de Man-Rogsa-Sharpe) medium containing glucose (for example, at 20 g/L), and 48 hours after the start of the culture, the number of lactic acid bacteria increases by a factor of 10 or more, and the pH decreases by 0.1 or more, compared with the start of the culture, it can be determined that the lactic acid bacteria have an excellent glucose-assimilating ability.

The KABP-067 strain further has an excellent glycogen-assimilating ability. As used herein, the "glycogen-assimilating ability" (glycogen-utilizing ability) of a lactic acid bacterium means the ability of the lactic acid bacterium to proliferate using glycogen as a nutrient source. When lactic acid bacteria have proliferated, lactic acid produced by the lactic acid bacteria decreases the surrounding pH. Accordingly, the glycogen-assimilating ability of a lactic acid bacterium such as the KABP-067 strain can be evaluated on the basis of the number and pH of the lactic acid bacteria in a liquid culture obtained by culturing the lactic acid bacteria in a glycogen-containing MRS medium. For example, in a case where lactic acid bacteria are cultured in an MRS medium containing glycogen (for example, at 20 g/L) instead of glucose, and 48 hours after the start of the culture, the number of lactic acid bacteria increases by a factor of 10 or more, and the pH decreases by 0.1 or more, compared with the start of the culture, it can be determined that the lactic acid bacteria have an excellent glycogen-assimilating ability.

In a case where the KABP-067 strain having a glycogen-assimilating ability is applied intravaginally, the KABP-067 strain proliferates using vaginal epithelial cell-derived glycogen as a nutrient source, produces lactic acid to decrease the intravaginal pH, thereby inhibiting the proliferation of other bacteria and fungi, and thus can bring about a vaginal microbiota having genus *Lactobacillus* bacteria dominantly. Furthermore, the proliferation of the genus *Lactobacillus* bacteria and the inhibition the proliferation of a pathogen in a vagina may improve the microbiota of a tissue or an organ around the vagina (for example, a uterus, ovary, fallopian tube, vulva, or the like).

The KABP-067 strain can also inhibit the proliferation of a pathogen that induces an infection of a genital organ, examples of which pathogen include *Escherichia coli* (*E. coli*), *Staphylococcus aureus (S. aureus), Proteus mirabilis (P. mirabilis), Streptococcus agalactiae* (*S. agalactiae*), *Candida albicans* (*C. albicans*), *Gardnerella vaginalis* (*G. vaginalis*), and *Prevotella bivia (P. bivia).*

Whether a lactic acid bacterium such as the KABP-067 strain inhibits the proliferation of a pathogen can be evaluated, for example, using the halo method as demonstrated in the below-described Example 3. Alternatively, as demonstrated in the below-described Example 4, the evaluation can also be performed by culturing a pathogen in a medium containing a cell free culture supernatant of a lactic acid bacterium, and by examining the pathogen-proliferation-inhibiting action of the cell free culture supernatant. Without being bound by any theory, it is considered that the KABP-067 strain inhibits the proliferation of a pathogen by producing lactic acid to decrease the surrounding pH, and besides, secreting another antibacterial substance.

In addition, the KABP-067 strain has sensitivity to gentamycin, streptomycin, tetracycline, erythromycin, clindamycin, chloramphenicol, and ampicillin, which are antibiotics. Accordingly, the KABP-067 strain that has been administered in vivo can be removed with an antibiotic, if necessary. The sensitivity to an antibiotic can be evaluated using an ordinary method, and, for example, can be evaluated using a minimum inhibitory concentration (MIC) as an index. The minimum inhibitory concentration can be measured, for example, in accordance with ISO 10932: 2010.

The *Lactobacillus crispatus* strain to be used in the present invention may be a living bacterium or a killed bacterium. The strain is preferably a living bacterium because the strain that is a living bacterium can proliferate using glycogen as a nutrient source in a vagina in particular. The *Lactobacillus crispatus* strain can be cultured using an ordinary method. Examples of a medium that can be used to culture the *Lactobacillus crispatus* strain include a medium that contains: a sugar (glucose, glycogen, or the like); amino acid; vitamin; mineral (magnesium, manganese, or the like); lipid; agent for inhibiting the proliferation of a bacterium other than lactic acid bacteria (ammonium citrate, sodium acetate, or the like); or pH buffer (potassium hydrogen phosphate or the like), for example, an MRS medium. The culture may be performed under anaerobic conditions, for example, at a culture temperature of 30 to 40°C, for example, 37°C, at a pH of 3.5 to 6.0, for example, a pH of 4.0 to 5.0.

### 1-3. Effect

The *Lactobacillus crispatus* strain according to the present invention can improve a genital microbiota. By improving a genital microbiota, the *Lactobacillus crispatus* strain according to the present invention can also provide a treating or preventing effect on an infection of a genital organ, improvement of fertility, decrease in the risk of premature delivery, and enhancement of a live birth rate.

### 2. Application of Lactobacillus crispatus strain

The *Lactobacillus crispatus* strain according to the present invention can be used to improve a genital microbiota. Accordingly, the present invention provides an agent for improving a genital microbiota, the agent consisting of the *Lactobacillus crispatus* strain according to the present invention (in particular, the KABP-067 strain). The *Lactobacillus crispatus* strain according to the present invention (in particular, the KABP-067 strain) can also be used in production of an agent for improving a genital microbiota.

As used herein, the "agent for improving a genital microbiota" means an agent having the effect of improving the microbiota of a genital organ. As used herein, the "genital organ" encompasses a male genital organ and a female genital organ, and is preferably a female genital organ. The "female genital organ" encompasses, for example, a vagina, uterus, ovary, fallopian tube, and vulva. As used herein, "improving a microbiota" encompasses increasing the number and/or ratio of the genus *Lactobacillus* bacteria in the microbiota, and decreasing the number and/or ratio of pathogens in the microbiota. The agent for improving a genital microbiota according to the present invention can be an agent that improves, for example, a vaginal microbiota.

### 3. Composition

### 3-1. Outline

The present invention relates to a composition comprising the above-described *Lactobacillus crispatus* strain according to the present invention.

### 3-2. Applications

The composition according to the present invention can be used to improve a genital microbiota, for example, to improve the microbiota of a female genital organ, for example, a vagina, uterus, ovary, fallopian tube, or vulva, preferably to improve the vaginal microbiota. The composition according to the present invention can also be used for vaginal cleaning. As used herein, "vaginal cleaning" encompasses washing off intravaginal waste products, and decreasing the number of intravaginal pathogens. The composition according to the present invention can also be used for treatment or prevention of an infection. The infection can be an infection of a genital organ, in particular, for example, an infection of a female genital organ, for example, a vagina, uterus, ovary, fallopian tube, or vulva. Examples of the infection of a genital organ include vaginitis (for example, bacterial vaginosis), cervicitis, endometritis, oophoritis, salpingitis, vulvitis, candidiasis, trichomoniasis, gonorrhea, and chlamydial infection. The infection can be an infection that is caused, for example, by a bacterium and/or fungus, for example, a genus *Escherichia* bacterium, a genus *Staphylococcus* bacterium, a genus *Proteus* bacterium, a genus *Streptococcus* bacterium, a genus *Candida* fungus, a genus *Gardnerella* bacterium, a genus *Prevotella* bacterium, or a combination thereof. More specifically, the infection can be an infection that is caused, for example, by *E. coli, S. aureus, P. mirabilis, S. agalactiae, C. albicans, G. vaginalis, P. bivia,* or a combination thereof. The composition according to the present invention can also be used to improve fertility or decrease the risk of premature delivery. The composition according to the present invention can also be used to enhance a live birth rate.

### 3-3. Constitution

### (1) Essential effective component

The composition according to the present invention comprises the *Lactobacillus crispatus* strain according to the present invention (in particular, the KABP-067 strain) as an essential effective component. The *Lactobacillus crispatus* strain to be used for the composition according to the present invention may be a living bacterium or a killed bacterium, and is preferably a living bacterium.

The amount of the *Lactobacillus crispatus* strain in the composition according to the present invention depends on the conditions such as the application, the subject of use, the using method, and the form of the composition, and can be an amount effective to improve a genital microbiota. The amount of the *Lactobacillus crispatus* strain in the composition according to the present invention can be, for example, but is not limited to, 0.01 wt% to 99 wt%, 0.01 wt% to 90 wt%, 0.01 wt% to 80 wt%, 0.01 wt% to 70 wt%, 0.01 wt% to 65 wt%, 0.01 wt% to 60 wt%, 0.01 wt% to 55 wt%, 0.01 wt% to 50 wt%, 0.01 wt% to 45 wt%, 0.01 wt% to 40 wt%, 0.01 wt% to 35 wt%, 0.01 wt% to 30 wt%, 0.01 wt% to 25 wt%, 0.01 wt% to 20 wt%, 0.01 wt% to 15 wt%, 0.01 wt% to 10 wt%, 0.01 wt% to 8 wt%, 0.01 wt% to 6 wt%, 0.01 wt% to 4 wt%, 0.01 wt% to 2 wt%, 0.01 wt% to 1 wt%, 0.01 wt% to 0.5 wt%, 0.01 wt% to 0.1 wt%, 0.01 wt% to 0.08 wt%, 0.01 wt% to 0.06 wt%, or 0.01 wt% to 0.04 wt%. The amount of the *Lactobacillus crispatus* strain in the composition according to the present invention can be, for example, but is not limited to, 10³ to 10¹³ bacteria/g, 10⁶ to 10¹¹ bacteria/g, 10⁸ to 10¹⁰ bacteria/g, or 10⁹ to 10¹⁰ bacteria/g.

### (2) Another component

The composition according to the present invention can further comprise one or more other effective components having the same pharmacologic action and/or different pharmacologic actions, unless they affect the genital microbiota improving action of the *Lactobacillus crispatus* strain according to the present invention.

The composition according to the present invention can also further comprise a non-effective component, for example, a carrier (solid carrier, liquid carrier, or the like), excipient, surfactant, emulsifier, binder, disintegrator, lubricant, solubilizer, suspending agent, coating agent, coloring agent, corrective, preservative, stabilizer, isotonizing agent, chelator, viscolizer, thickener, buffer, pH adjustor, or the like, unless it affects the genital microbiota improving action of the *Lactobacillus crispatus* strain according to the present invention.

### 3-4. Form

The composition according to the present invention may be formulated in any dosage form such as a solid preparation such as a tablet, granule, powder, pill, or capsule; a liquid preparation such as a liquid agent, suspension, or syrup; an injection having a solid preparation or a liquid preparation encapsulated therein; gel; or a spray, but is not limited thereto.

### 3-5. Subject of administration

A subject of administration (test subject) of the composition according to the present invention may be any of a male human, male animal, female human, and female animal, and is preferably a female human or a female animal. A subject of administration of the composition according to the present invention is any mammal, for example, a human, domestic animal (horse, cow, sheep, goat, pig, or the like), pet animal (dog, cat, rabbit, or the like), or laboratory animal (mouse, rat, monkey, or the like), and is preferably a human. A subject of administration of the composition according to the present invention may be, for example, a subject having a microbiota comprising genus *Lactobacillus* bacteria at a small ratio (for example, less than 90%), or a subject having an infection in the genital organ.

### 3-6. Mode of administration

The composition according to the present invention can be orally administered or parenterally administered, but is preferably administered to a genital organ (for example, a female genital organ, for example, a vagina, uterus, ovary, fallopian tube, or vulva). The composition according to the present invention can be preferably a composition for administration to a genital organ, more preferably a composition for intravaginal administration. The composition according to the present invention may be administered in a single dose, or may be administered in multiple doses at intervals of several hours to several months.

### 4. Method of improving genital microbiota

The present invention relates to a method of improving a genital microbiota. The method of improving a genital microbiota according to the present invention comprises an administration step of administering the *Lactobacillus crispatus* strain according to the present invention (in particular, the KABP-067 strain) or the composition according to the present invention to a subject. In the method of improving a genital microbiota according to the present invention, a dose, a subject of administration, and a mode of administration are as described in "3. Composition" above. The method of improving a genital microbiota according to the present invention can be a method of improving, for example, a microbiota of a female genital organ, for example, a vagina, uterus, ovary, fallopian tube, or vulva, particularly a method of improving a vaginal microbiota.

### 5. Method of treating or preventing infection

The present invention relates to a method of treating or preventing an infection. The method of treating or preventing an infection according to the present invention comprises an administration step of administering the *Lactobacillus crispatus* strain according to the present invention (in particular, the KABP-067 strain) or the composition according to the present invention to a subject. In the method of treating or preventing an infection according to the present invention, a dose, a subject of administration, and a mode of administration are as described in "3. Composition" above. An infection as a subject of the method of treating or preventing an infection according to the present invention can be, in particular, an infection of a genital organ, for example, an infection of a female genital organ, for example, a vagina, uterus, ovary, fallopian tube, or vulva. The infection can be, for example, vaginitis (for example, bacterial vaginosis), cervicitis, endometritis, oophoritis, salpingitis, vulvitis, candidiasis, trichomoniasis, gonorrhea, or chlamydial infection. The infection can be an infection that is caused, for example, by a bacterium and/or fungus, for example, a genus *Escherichia* bacterium, a genus *Staphylococcus* bacterium, a genus *Proteus* bacterium, a genus *Streptococcus* bacterium, a genus *Candida* fungus, a genus *Gardnerella* bacterium, a genus *Prevotella* bacterium, or a combination thereof. More specifically, the infection can be an infection that is caused, for example, by *E. coli, S. aureus, P. mirabilis, S. agalactiae, C. albicans, G. vaginalis, P. bivia,* or a combination thereof.

### 6. Method of improving fertility

The present invention relates to a method of improving fertility. The method of improving fertility according to the present invention comprises an administration step of administering the *Lactobacillus crispatus* strain according to the present invention (in particular, the KABP-067 strain) or the composition according to the present invention to a subject. In the method of improving fertility according to the present invention, a dose, a subject of administration, and a mode of administration are described in "3. Composition" above.

### 7. Method of decreasing risk of premature delivery

The present invention relates to a method of decreasing the risk of premature delivery. The method of decreasing the risk of premature delivery according to the present invention comprises an administration step of administering the *Lactobacillus crispatus* strain according to the present invention (in particular, the KABP-067 strain) or the composition according to the present invention to a subject. In the method of decreasing the risk of premature delivery according to the present invention, a dose, a subject of administration, and a mode of administration are described in "3. Composition" above.

### 8. Method of enhancing live birth rate

The present invention relates to a method of enhancing a live birth rate. The method of enhancing a live birth rate according to the present invention comprises an administration step of administering the *Lactobacillus crispatus* strain according to the present invention (in particular, the KABP-067 strain) or the composition according to the present invention to a subject. In the method of enhancing a live birth rate according to the present invention, a dose, a subject of administration, and a mode of administration are as described in "3. Composition" above.

### EXAMPLES

The present invention will be more specifically described below with reference to Examples. However, the technical scope of the present invention should not be limited to these Examples.

In the following Examples 1 to 5, screening for genus *Lactobacillus* bacteria suitable for improvement of a genital microbiota were performed, on the basis of a proliferative ability, glycogen-assimilating ability, pathogen-inhibiting effect, and antibiotic sensitivity.

### <Example 1: Proliferative ability>

### (Outline)

In this Example, genus *Lactobacillus* bacteria (*L. crispatus*) having a high proliferation rate and an ability to decrease the pH was selected.

### (Method and results)

From colonies isolated from the vagina of healthy Italian females, 254 *L. crispatus* strains were collected. From these strains, duplicated strains according to their RAPD (random amplified polymorphic DNA) profile and previously known strains were excluded, and 26 strains were selected. The selected strains were stationarily cultured in a commercially available MRS medium (BD, Difco^{™} Lactobacilli MRS Broth, 288130) under anaerobic conditions at 37°C for 24 hours. The OD600 of the liquid culture was measured 24 hours after the start of the stationary culture to evaluate the proliferative ability of each strain. As a result, it was shown that 14 strains (KABP-067, Comparative Strains 1, 2, 3, 5, 6, 7, 12, 17, 18, 20, 21, 23, and 24) out of the 26 strains have a high proliferation rate, and can decrease the pH. In the following Example 2, these 14 *L. crispatus* strains were further evaluated.

### <Example 2: Glycogen-assimilating ability>

### (Outline)

In this Example, the *L. crispatus* strains having a glycogen-assimilating ability were selected.

### (Material)

### Glucose-containing MRS medium

20 g/L D-(+) glucose, 1 g/L Tween 80, 2 g/L diammonium hydrogencitrate, 5 g/L sodium acetate, 0.1 g/L magnesium sulfate heptahydrate, 0.05 g/L manganese sulfate monohydrate, 2 g/L dipotassium hydrogenphosphate, 10 g/L Bacto^{™} proteose pepton (Gibco, Catalog No.: 211693), 10 g/L Difco^{™} Beaf extract (Gibco, Catalog No.: 212610), and 5 g/L BD Difco^{™} Yeast extract (BD, SKU: 239210) were dissolved in distilled water, and the resultant was adjusted to a pH of 4.5 using lactic acid. Then, the resulting solution was sterilized in an autoclave to prepare a glucose-containing MRS medium.

### Glycogen-containing MRS medium

20 g/L glycogen (oyster-derived glycogen, 072-05562, Fujifilm Wako Pure Chemical Corporation), 1 g/L Tween 80, 2 g/L diammonium hydrogencitrate, 5 g/L sodium acetate, 0.1 g/L magnesium sulfate heptahydrate, 0.05 g/L manganese sulfate monohydrate, 2 g/L dipotassium hydrogenphosphate, 10 g/L Bacto^{™} proteose pepton (Gibco, Catalog No.: 211693), 10 g/L Difco^{™} Beaf extract (Gibco, Catalog No.: 212610), and 5 g/L BD Difco^{™} Yeast extract (BD, SKU: 239210) were dissolved in distilled water, and the resultant was adjusted to a pH of 4.5 using lactic acid. Then, the resulting solution was sterilized in an autoclave to prepare a glycogen-containing MRS medium.

### (Method)

The glycogen-assimilating ability and glucose-assimilating ability of each of the 14 *L. crispatus* strains (KABP-067, Comparative Strain 1, 2, 3, 5, 6, 7, 12, 17, 18, 20, 21, 23 and 24) selected in Example 1 were measured. First, each strain was inoculated at 2 vol% in 5 mL of a commercially available MRS medium (BD, Difco^{™} Lactobacilli MRS Broth, 288130), and stationarily cultured in a 15 mL centrifuge tube under anaerobic conditions at 37°C (the G1 culture). Then, each strain was washed with PBS to remove the liquid culture components. Then, each strain was re-suspended in a glucose-containing MRS medium or glycogen-containing MRS medium prepared in (Material) above, and inoculated in a 5 mL MRS medium in such a manner that the OD600 was 0.1 (the number of bacteria, 2 × 10⁸ bacteria/mL). Then, each strain was stationarily cultured in a 15 mL centrifuge tube under anaerobic conditions at 37°C (the G2 culture). 0 hours, 24 hours, and 48 hours after the start of the culture, samples were taken, and the OD600 and the pH were measured.

### (Results)

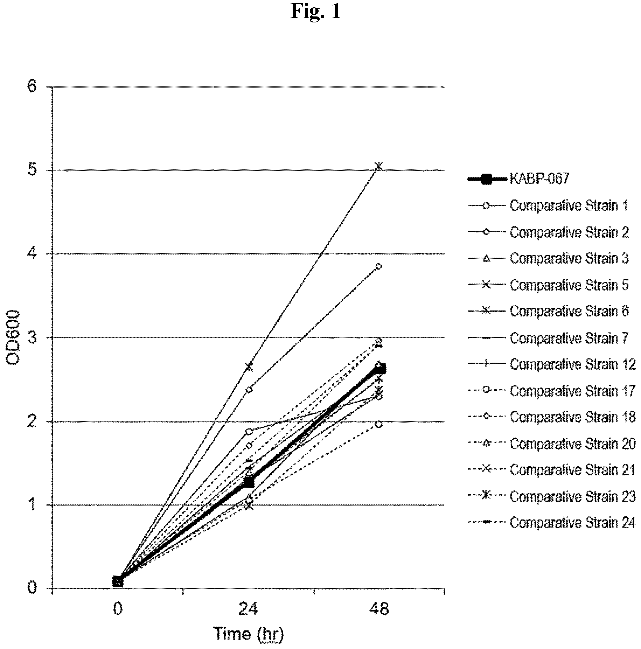

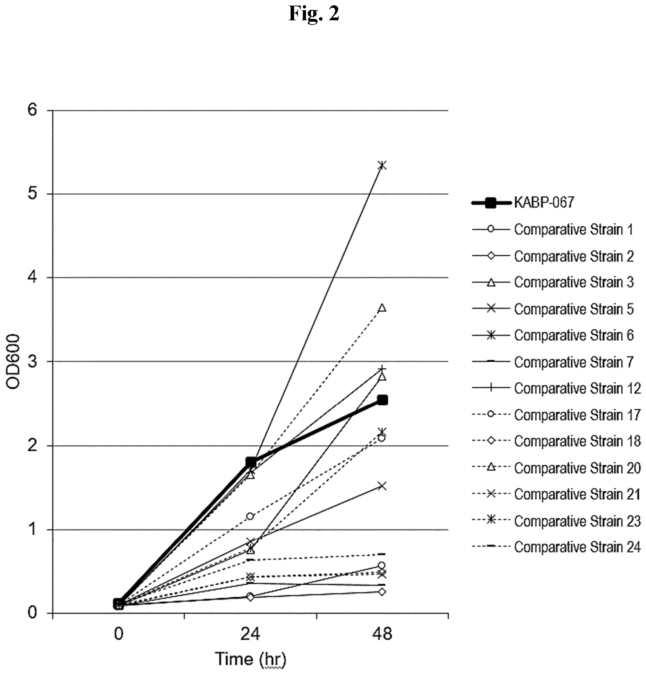

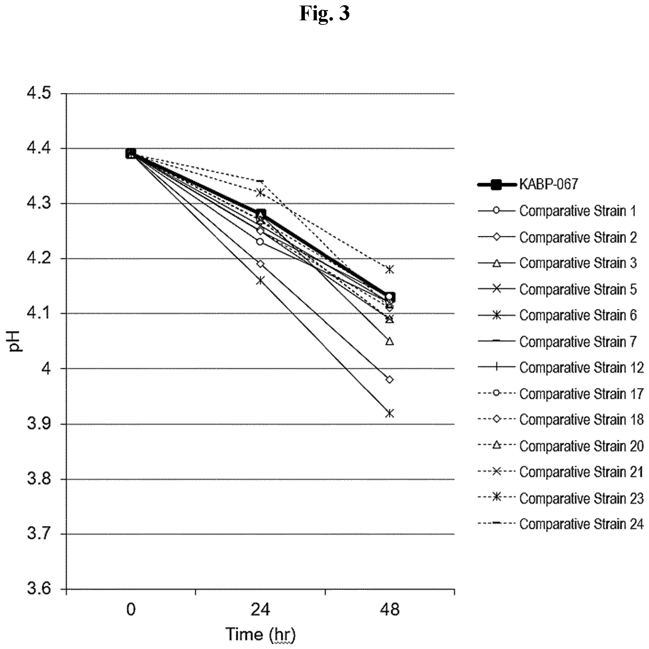

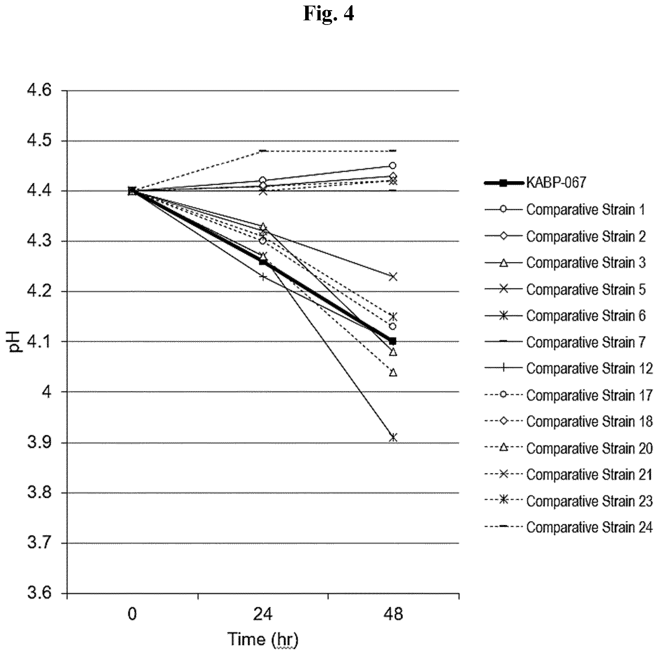

The Results are shown in FIG. 1 to FIG. 4. All the 14 *L. crispatus* strains (KABP-067, Comparative Strains 1, 2, 3, 5, 6, 7, 12, 17, 18, 20, 21, 23, and 24) had a high proliferative ability and pH-decreasing ability in the glucose-containing MRS medium (FIG. 1 and FIG. 3). These results indicate that any of the 14 strains has an excellent glucose-assimilating ability. In addition, eight strains (KABP-067, Comparative Strains 3, 5, 6, 12, 17, 20, and 23) of the 14 *L. crispatus* strains had a high proliferative ability and pH-decreasing ability also in the glycogen-containing MRS medium (FIG. 2 and FIG. 4). These results indicate that these eight strains have an excellent glycogen-assimilating ability. In the following Examples 3 and 4, the eight strains that exhibited a glycogen-assimilating ability were further evaluated.

### <Example 3: Pathogen-inhibiting effect (halo method)>

### (Outline)

In this Example, the *L. crispatus* strains capable of inhibiting the proliferation of *E. coli, S. aureus, P. mirabilis,* and *S. agalactiae* that are vaginitis-related bacteria were selected.

### (Method)

The pathogen-inhibiting effect of each of the eight *L. crispatus* strains (KABP-067, Comparative Strain 3, 5, 6, 12, 17, 20, and 23) selected in Example 2 was evaluated using the halo method. The pathogens used were: *Escherichia coli* (*E. coli*) DSM1103 strain and ATCC700414 strain; *Staphylococcus aureus* (*S. aureus*) DSM799 strain; *Proteus mirabilis* (*P. mirabilis*) DSM102257 strain; and *Streptococcus agalactiae* (*S. agalactiae*) DSM2134T strain. On a plate supplemented with pathogens (the bacterial concentration: 10⁸ CFU/mL), a soft agar disc (0.9% agar) containing 10% liquid culture obtained by culturing an *L. crispatus* strain overnight was placed. Then, they were incubated at 37°C for 48 hours. After that, the radius of a halo that appeared was measured.

### (Results)

The Results are shown in Table 1.

**[Table 1]**

| ***L. crispatus* strain** | **Target pathogen** | | | | |
|---|---|---|---|---|---|
| | ***E. coli* DSM 1103** | ***E. coli* ATCC 700414** | ***S. aureus* DSM 799** | ***P. mirabilis* DSM 102257** | ***S. agalactiae* DSM 2134T** |
| KABP-067 | + | ++ | + | + | + |
| Comparative Strain 3 | + | ++ | + | + | + |
| Comparative Strain 5 | + | + | + | + | + |
| Comparative Strain 6 | + | ++ | + | + | + |
| Comparative Strain 12 | + | + | + | + | + |
| Comparative Strain 17 | + | ++ | + | + | + |
| Comparative Strain 20 | + | + | + | + | + |
| Comparative Strain 23 | + | + | + | + | + |

| | | | | | |
|---|---|---|---|---|---|
| + denotes that the radius of the halo was 1 mm to 10 mm. ++ denotes that the radius of the halo was 11 mm to 19 mm. +++ denotes that the radius of the halo was 20 mm or more. | | | | | |

As shown in Table 1, any of the 8 *L. crispatus* strains (KABP-067, Comparative Strains 3, 5, 6, 12, 17, 20, and 23) inhibited the proliferation of all of the pathogens (*E. coli, S. aureus, P. mirabilis,* and *S. agalactiae*) tested.

### <Example 4: Pathogen-inhibiting effect (liquid inhibition method)>

### (Outline)

In this Example, the *L. crispatus* strains having an inhibitory effect on yeasts of genus *Candida,* and bacteria of genus *Gardnerella,* and genus *Prevotella* that are vaginitis-related microorganisms were selected.

### (Method)

The pathogen-inhibiting effect of each of the eight *L. crispatus* strains (KABP-067, Comparative Strain 3, 5, 6, 12, 17, 20, and 23) selected in Example 2 was evaluated using the liquid inhibition method. The pathogens used were the *Candida albicans* (*C. albicans*) DSM1386 strain, *Candida glabrata* (*C. glabrata*) DSM11226 strain, *Gardnerella vaginalis* (*G. vaginalis*) DSM4944T strain, and *Prevotella bivia* (*P. bivia*) DSM20514T strain. The activated liquid culture of the *L. crispatus* strain grown for 16 h was prepared. Cell free culture supernatant was obtained from the activated liquid culture by centrifugation and treatment through a 0.22 µm filter to remove bacteria. The activated liquid culture of the pathogen grown for 16 h was obtained. Pathogen culture was standardized to a cell concentration equal to McFarland 0.5. Next, 2.5 mL of the cell free culture supernatant and 2.5 mL of the standardized pathogen liquid culture were mixed reaching a final concentration of 10³ CFU/mL, and pathogen was cultured in tubes at 37°C. As a control, the pathogen liquid culture without the cell free culture supernatant was cultured in the same manner. After 24 hours, the OD625 was measured on a plate reader, and the proliferation-inhibiting rate (%) was calculated as follows: {(OD625 of control - OD625 of the pathogen liquid culture containing the cell-free culture supernatant) / OD625 of control} × 100.

### (Results)

The Results are shown in Table 2.

**[Table 2]**

| ***L. crispatus* strain** | **Target pathogen** | | | |
|---|---|---|---|---|
| | ***C. albicans* DSM 1386** | ***C. glabrata* DSM 11226** | ***G. vaginalis* DSM 4944T** | ***P. bivia* DSM 20514T** |
| KABP-067 | ++ | - | + | ++ |
| Comparative Strain 3 | - | - | + | + |
| Comparative Strain 5 | ++ | + | + | ++ |
| Comparative Strain 6 | - | - | + | + |
| Comparative Strain 12 | - | - | + | + |
| Comparative Strain 17 | - | - | + | + |
| Comparative Strain 20 | - | - | + | ++ |
| Comparative Strain 23 | - | - | + | + |

| | | | | |
|---|---|---|---|---|
| - denotes that the proliferation-inhibiting rate was less than 1%. + denotes that the proliferation-inhibiting rate was 1% or more and less than 30%. ++ denotes that the proliferation-inhibiting rate was 30% or more and less than 60%. | | | | |

As shown in Table 2, among the eight *L. crispatus* strains, two strains (KABP-067 and Comparative Strain 5) inhibited the proliferation of genus *Candida* yeasts, genus *Gardnerella* bacteria, and genus *Prevotella* bacteria. The other six strains inhibited the proliferation of bacteria of genus *Gardnerella* bacteria and genus *Prevotella* bacteria, but did not inhibit the proliferation of genus *Candida* yeasts. In the following Example 5, KABP-067 and Comparative Strain 5 were further evaluated.

### (Example 5) Antibiotic sensitivity

### (Outline)

In this Example, the *L. crispatus* strain having sensitivity to antibiotics was selected.

### (Method)

The antibiotic sensitivity of each of two *L. crispatus* strains (KABP-067 and Comparative Strain 5) selected in Example 4 was evaluated. The antibiotic sensitivity was tested in accordance with the method in ISO 10932: 2010, using gentamycin, kanamycin, streptomycin, tetracycline, erythromycin, clindamycin, chloramphenicol, and ampicillin that are the eight kinds of antibiotics required by EFSA (Europe Food Safety Authority). The antibiotic sensitivity was determined by comparing a minimum inhibitory concentration (MIC). The cutoff value used was a cutoff value defined by EFSA. As a control, the antibiotic sensitivity of a *L. paracasei* ATCC 334 strain was evaluated in the same manner.

### (Results)

The Results are shown in Table 3.

**[Table 3]**

| | Gentamycin | | Kanamycin | | Streptomycin | | Tetracycline | | Erythromycin | | Clindamycin | | Chloramphenicol | | Ampicillin | | |
|---|---|---|---|---|---|---|---|---|---|---|---|---|---|---|---|---|---|
| *Cut-off (EFSA 2018)* | 16 | | 16 | | 16 | | 4 | | 1 | | 4 | | 4 | | 2 | | |
| | R1 | R2 | R1 | R2 | R1 | R2 | R1 | R2 | R1 | R2 | R1 | R2 | R1 | R2 | R1 | R2 | #resistances |
| L. paracasei ATCC 334 (control) | 4 | 4 | 64 | 32 | 16 | 16 | 1 | 1 | 0.125 | 0.125 | 0.064 | 0.064 | 8 | 8 | 1 | 1 | |
| KABP-067 | 4 | 4 | 64 | 64 | 8 | 8 | 2 | 4 | <0.016 | 0.032 | 0.064 | 0.032 | 2 | 2 | 2 | 1 | 1 |
| Comparative Strain 5 | 16 | 16 | 256 | 256 | 64 | 64 | 2 | 2 | 0.064 | 0.064 | 0.064 | 0.128 | 4 | 4 | 1 | 1 | 2 |

As shown in Table 3, KABP-067 exhibited sensitivity to seven kinds of antibiotics (gentamycin, streptomycin, tetracycline, erythromycin, clindamycin, chloramphenicol, and ampicillin), but not to kanamycin. Comparative Strain 5 exhibited sensitivity to six kinds of antibiotics (gentamycin, tetracycline, erythromycin, clindamycin, chloramphenicol, and ampicillin), but not to kanamycin and streptomycin. The MIC of KABP-067 was generally smaller than the MIC of Comparative Strain 5, and thus, it was revealed that KABP-067 exhibits a higher antibiotic sensitivity.

## Claims

1. A *Lactobacillus crispatus* strain deposited under accession No. CECT31133.

2. A composition comprising a living bacterium of the *Lactobacillus crispatus* strain according to claim 1.

3. The composition according to claim 2, wherein said composition is suitable for intravaginal administration.

4. The composition according to claim 2 or 3, for use in improvement of a genital microbiota.

5. The composition according to claim 2 or 3, for use in vaginal cleaning.

6. The composition according to claim 2 or 3, for use in prevention or treatment of an infection.

7. The composition according to claim 2 or 3, for use in improvement of fertility.

8. The composition according to claim 2 or 3, for use in decreasing a risk of premature delivery.
